# EUROPEAN PATENT SPECIFICATION

(11) **EP 1 941 274 B1**
(45) Date of publication and mention of the grant of the patent: **19.08.2015**
(21) Application number: 06791942.3
(22) Date of filing: 08.09.2006
(51) Int. Cl.: G01N 33/53, G01N 33/68, G01N 33/92, C07K 14/47

(54) **METHOD FOR PREDICTING THE PROGRESSION OF CHRONIC KIDNEY DISEASE BY MEASURING APOLIPOPROTEIN A-IV**
VERFAHREN ZUR VORHERSAGE DES FORTSCHREITENS EINER CHRONISCHEN NIERENKRANKHEIT DURCH MESSEN VON APOLIPOPROTEIN A-IV
MÉTHODE DE PRÉDICTION DE LA PROGRESSION D'UNE MALADIE CHRONIQUE DES REINS PAR LE DOSAGE DE L'APOLIPOPROTEINE A-IV

(30) Priority: 09.09.2005 EP 05019668
(43) Date of publication of application: 09.07.2008
(73) Proprietor: Medizinische Universität Innsbruck, 6020 Innsbruck (AT)
(72) Inventor: KRONENBERG, Florian, A-6020 Innsbruck (AT)
(74) Representative: Krauss, Jan
(86) International application number: PCT/EP2006/008785
(87) International publication number: WO 2007/028636

(56) References cited:
- WO-A2-03/010544
- DATABASE MEDLINE [Online] US NATIONAL LIBRARY OF MEDICINE (NLM), BETHESDA, MD, US; 1996, ROCCO M V ET AL: "Measurement of glomerular filtration rate using nonradioactive Iohexol: comparison of two one-compartment models." XP002408201 Database accession no. NLM8919230 & AMERICAN JOURNAL OF NEPHROLOGY. 1996, vol. 16, no. 2, 1996, pages 138-143, ISSN: 0250-8095
- DATABASE MEDLINE [Online] US NATIONAL LIBRARY OF MEDICINE (NLM), BETHESDA, MD, US; 15 June 2005 (2005-06-15), HSU CHARLES C ET AL: "Apolipoprotein E and progression of chronic kidney disease." XP002408202 Database accession no. NLM15956634 cited in the application & JAMA : THE JOURNAL OF THE AMERICAN MEDICAL ASSOCIATION 15 JUN 2005, vol. 293, no. 23, 15 June 2005 (2005-06-15), pages 2892-2899, ISSN: 1538-3598
- DIEPLINGER H ET AL: "PLASMA APOLIPOPROTEIN A-IV METABOLISM IN PATIENTS WITH CHRONIC RENAL DISEASE" EUROPEAN JOURNAL OF CLINICAL INVESTIGATION, BLACKWELL SCIENTIFIC PUBLICATIONS, vol. 22, no. 3, 1992, pages 166-174, XP009008717 ISSN: 0014-2972 cited in the application
- VERGES B: "APOLIPROTEIN A-IV IN DIABETES MELLITUS//APOLIPOPROTEINE A-IV AU COURS DU DIABETE", DIABETE & METABOLISME, MASSON, PARIS, FR, vol. 21, no. 2, 1 January 1995 (1995-01-01), pages 99-105, XP009005610, ISSN: 0338-1684
- DATABASE MEDLINE [Online] US NATIONAL LIBRARY OF MEDICINE (NLM), BETHESDA, MD, US NLM18577393, 03 September 2008 SHEN LING ET AL: 'Characterization of apolipoprotein A-IV in brain areas involved in energy homeostasis.' Database accession no. NLM18577393

## Description

The present invention refers to a method for predicting chronic kidney disease progression, preferably for predicting a disease progression in a subject having the disease manifested, comprising a determination of apolipoprotein A-IV (ApoA-IV) in a sample, preferably comprising a determination of apoA-IV concentration in serum. The present invention further refers to an *in vitro* use of ApoA-IV as a predictor of chronic kidney disease progression.

### Background of the Invention

Without treatment many kidney diseases tend to progress to renal failure. However, the rate of progression shows considerable interindividual variability and is dependent on multiple factors. Moorhead and colleagues had proposed in the early eighties that abnormalities in lipoprotein metabolism cause glomerular and tubulointerstitial damage promoting progression of chronic kidney disease *⁽¹⁾*. Experimental studies supported this concept that lipids contribute to progressive renal damage *⁽²⁾* by causing macrophage activation and infiltration in the kidney with resultant tubulointerstitial and endothelial cell injury. However, the extent, if any, to which dyslipidemia contributes to progression of renal disease in humans and the possible mechanisms by which this may occur have remained unclear *⁽³⁾*. Data from the population-based ARIC study support a role of altered lipid metabolism in developing renal dysfunction *⁽⁴⁾.* Only few prospective studies in patients with primary non-diabetic kidney disease, however, have addressed this question *⁽⁵⁻¹²⁾* and the results have remained ambiguous.

To establish and characterize a relationship between dyslipidemia and renal risk long-term prospective follow-up studies with a reliable measurement of kidney function at baseline and well defined renal function endpoints are requested *⁽¹³⁾*. A better understanding of said relationship may allow a prediction of the progression of chronic kidney disease.

It was therefore an object of the present invention to provide an improved method for predicting chronic kidney disease progression.

WO03010544 is a cross sectional study showing that an increased serum concentration of ApoA-IV is observed already at an early stage of kidney disease. The serum level of Apo-AIV is inversely correlated to the glomerular filtration rate and kidney functioning.

### Summary of the Invention

The object of the present invention is solved by a method for predicting chronic kidney disease progression in a subject to be examined, wherein the method comprises a determination of apolipoprotin A-IV (ApoA-IV) in a sample taken from the subject to be examined according to claim 1.

In one embodiment, the subject is at risk of developing a chronic kidney disease.

In a preferred embodiment, the subject is diagnosed as having a primary kidney disease.

In an alternative embodiment, the subject is diagnosed as having a chronic kidney disease developed from a primary kidney disease.

In one embodiment, the disease is a non-diabetic chronic kidney disease and the primary kidney disease is a non-diabetic disease.

In a preferred embodiment, the primary kidney disease is selected from the group consisting of glomerulonephritis, adult polycystic kidney disease, and interstitial nephritis.

In an alternative embodiment, the disease is a diabetic chronic kidney disease, preferably diabetic nephropathy.

In one embodiment, the subject is a vertebrate, more preferably a mammal, most preferably a human.

The sample is a bodily fluid sample.

The bodily fluid is preferably selected from the group consisting of whole blood, plasma and serum.

In a particularly preferred embodiment, the sample is serum.

In one embodiment, the determination of ApoA-IV is a quantitative measurement of ApoA-IV concentration.

In a preferred embodiment, the ApoA-IV concentration is determined in serum by use of an enzyme-linked immunosorbent assay (ELISA).

The ApoA-IV concentration is compared to a reference according to claim 1..

In a preferred embodiment, the reference is an ApoA-IV concentration determined in a sample obtained from the subject to be examined at an earlier time.

An elevation of the ApoA-IV concentration is a predictor of disease progression.

In a preferred embodiment, an elevation of the ApoA-IV concentration of at least 3 mg/dl is a predictor of disease progression.

In a particular preferred embodiment, an elevation of the ApoA-IV concentration of at least 5 mg/dl is a predictor of disease progression.

In a particular preferred embodiment, an elevation of the ApoA-IV concentration of at least 10 mg/dl is a predictor of disease progression.

In one embodiment, the method further comprises a determination of the glomerular filtration rate (GFR).

In a preferred embodiment, the GFR is determined by use of the iothalamate clearance technique.

In another preferred embodiment, a reduction of the GFR is a predictor of disease progression.

In a particularly preferred embodiment, a reduction of the GFR of at least 10 ml/min/1.73 m² is a predictor of disease progression.

In one embodiment, the method further comprises a determination of the apolipoprotein E (apoE) genotype.

In a preferred embodiment, the apoE genotype is determined by use of a 5' nuclease allelic discrimination assay.

In one embodiment, the apoE genotype ε2/ε4 is a predictor of increased disease progression risk.

The object of the present invention is further solved by use of a method for predicting chronic kidney disease according to the present invention.

The object of the present invention is further solved by in vitro use of ApoA-IV as a predictor of chronic kidney disease progression according to claim 8.

In one embodiment, the disease is a non-diabetic chronic kidney disease.

In a preferred embodiment, the non-diabetic chronic kidney disease has developed from a primary kidney disease, selected from the group consisting of glomerulonephritis, adult polycystic kidney disease, and interstitial nephritis.

In one embodiment, the disease is a diabetic chronic kidney disease.

In one embodiment, ApoA-IV is used together with GFR as predictors of chronic kidney disease progression.

In an alternative embodiment, ApoA-IV is used together with ApoE genotype ε2/ε4 as predictors of chronic kidney disease progression.

In a preferred embodiment, ApoA-IV is used together with GFR and ApoE genotype ε2/ε4 as predictors of chronic kidney disease progression.

The term "chronic kidney disease progression" is defined as doubling a baseline serum creatinine and/or terminal real failure necessitating renal replacement therapy.

The term "determination of ApoA-IV" comprises both quantifying ApoA-IV concentration and qualitatively detecting ApoA-IV in a sample. In case that an ApoA-IV concentration is quantified, the amount of ApoA-IV in a sample of know volume is measured, and then the result obtained is connected to the sample volume.

It has not been firmly established whether dyslipidemia contributes independently to the progression of kidney disease. It was therefore the aim of the present prospective seven year study in a cohort of non-diabetic patients with mild to moderate primary kidney disease to evaluate the predictive value of lipoproteins for the progression of kidney disease. We assessed lipid and lipoprotein parameters including levels of total, HDL and LDL cholesterol, triglycerides, lipoprotein(a), apolipoprotein A-IV and the apolipoprotein E polymorphism in 177 patients with mild to moderate renal insufficiency who were prospectively followed for up to seven years. Progression of kidney disease was defined as doubling of baseline serum creatinine and/or terminal renal failure necessitating renal replacement therapy. In univariate analysis, patients who reached a progression endpoint (n=65) were significantly older and had higher serum creatinine and proteinuria as well as lower GFR and haemoglobin levels. In addition, baseline apolipoprotein A-IV and triglyceride concentrations were higher and HDL cholesterol levels lower. Multivariate Cox regression analysis revealed that baseline GFR (hazard ratio 0.714; 95%CI 0.627-0.814 for an increment of 10 mL/min/1.73m²; p<0.0001) and serum apolipoprotein A-IV concentrations (hazard ratio 1.062; 95%CI 1.018-1.108 for an increment of 1 mg/dL; p=0.006) were significant predictors of disease progression. Patients with apolipoprotein A-IV levels above the median had a significantly faster progression (p<0.0001), and their mean follow-up time to a progression endpoint was 53.7 (95%CI 47.6-59.8) months as compared to 70.0 (95%CI 64.6-75.4) months in patients with apolipoprotein A-IV levels below the median. For the apolipoprotein E polymorphism only the genotype ε2/ε4 was associated with an increased risk of progression.

In summary, our prospective study in patients with non-diabetic primary kidney disease demonstrated that apolipoprotein A-IV concentration is a novel independent predictor of progression. However, we found no significant and independent association between the classical lipid parameters and the progression of kidney disease to an endpoint. Our study failed to confirm an association between apoE polymorphism and progression of CKD that had been described in previous studies.

### Detailed Description of the Invention

The present invention shall now be described in more detail on the basis of the examples and by making reference to the tables and to the attached figures.
- Figure 1: shows Kaplan-Meier curves of renal endpoints in patients with infra- and supra-median plasma apolipoprotein A-IV (apoA-IV) concentrations. In patients with supra-median plasma apoA-IV concentrations (i.e. > 25.9 mg/dL) progression was significantly faster (log-rank test, p<0.0001).

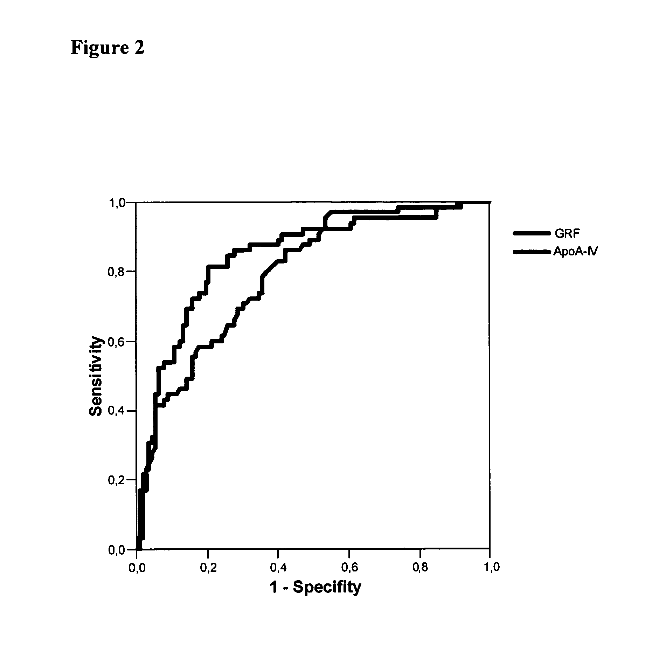

- Figure 2: shows a comparison of the methods for determining ApoA-IV and GFR with respect to sensitivity and specificity (ROC analysis). The results obtained demonstrate that the performance of ApoA-IV determination is similarly good as that of GFR determination.

### EXAMPLES

### EXAMPLE 1: Materials and Methods

### 1.1. Patients and baseline investigations

We examined at baseline 227 Caucasian patients aged between 18 and 65 years with non-diabetic chronic kidney disease (CKD) and various degrees of renal impairment. These patients were recruited from eight nephrology departments in Germany, Austria and South Tyrol as described earlier ^{*(14,15*)}. The study was approved by the institutional Ethic Committees, and all subjects gave written informed consent. They had stable renal function for at least 3 months before entry into the study. Exclusion criteria were treatment with immunosuppressive agents, fish oil or erythropoietin, serum creatinine above 6 mg/dL, diabetes mellitus of any type, malignancy, liver, thyroid or infectious disease, nephrotic syndrome (defined as proteinuria > 3.5 g/1.73 m²/day), organ transplantation, allergy to ionic contrast media and pregnancy. The primary cause of kidney disease was glomerulonephritis in 97 (biopsy-confirmed in 90) patients, adult polycystic kidney disease in 37 patients, interstitial nephritis in 24 patients, other types of kidney disease in 43 patients and unknown in 26 patients.

In order to avoid interobserver differences, all patients were recruited by one physician who visited all participating centers. Patient history, including smoking habits and antihypertensive treatment at baseline, was recorded by interview and confirmed by checking patient records. This was complemented by clinical examination including assessment of body mass index (BMI) and blood pressure. Hypertension was defined as blood pressure above 140/90 mm Hg and/or the use of antihypertensive medication. We also calculated pulse pressure as the difference between systolic and diastolic blood pressure. Antihypertensive medication (if present) was withheld on the day of the study in order to minimize interference with measurements of the glomerular filtration rate (GFR). Antihypertensive drugs were taken by 179 patients (79%): diuretics (n=83; 37%), ACE-inhibitors (n=123; 54%), calcium channel blockers (n=78; 34%), beta receptor blockers (n=67; 30%) and alpha-1 receptor blockers (n=35; 16%).

### 1.2. Prospective follow-up

After the baseline investigation patients were followed prospectively until the primary study endpoint or the end of the observation period was reached. The primary endpoint was defined as doubling of baseline serum creatinine and/or terminal renal failure necessitating renal replacement therapy.

A total of 177 patients (78%) from the baseline cohort could be assessed during the follow-up. Patients lost to follow-up (n=50) had significantly better renal function than patients not lost for follow-up, i.e. a higher mean GFR (91±44 vs. 64±39 mL/min/1.73m²; p<0.01). However, both groups did not differ significantly with respect to age and gender. Patients lost to follow-up had moved away or were not referred by their private physicians for follow-up visits in the renal units.

### 1.3. Laboratory measurements

Blood samples for measurement of routine chemistry, high sensitivity C-reactive protein (hsCRP) and lipid parameters were taken after an overnight fast of at least 12 hours. The samples were immediately centrifuged at 1.500 g and 4°C for 10 minutes, and the supernatants stored in aliquots at -80°C until further use.

Serum apolipoprotein A-IV (apoA-IV) concentrations were determined with an enzyme-linked immunosorbent assay (ELISA) *⁽¹⁶⁾*. Lp(a) quantification and apo(a) phenotyping were performed as described in detail *⁽¹⁴⁾* with a double-antibody ELISA and by SDS-agarose gel electrophoresis, respectively. Measurements of serum albumin, total and HDL cholesterol, triglycerides and hsCRP were performed using routine laboratory tests. LDL cholesterol was calculated according to the Friedewald formula. In addition, GFR was assessed in all patients using the iothalamate clearance technique as described in detail elsewhere *⁽¹⁷⁾*. Apolipoprotein E (apoE) genotyping was performed with 5' nuclease allelic discrimination (Taqman) assays.

### 1.4. Statistical analysis

Statistical analysis was performed with Statistical Package for the Social Sciences (SPSS) for Windows 12.01. Univariate comparisons of continuous variables between various groups were performed using an unpaired t test or the nonparametric Wilcoxon rank sum test in case of non-normally distributed variables. Dichotomized variables were compared using Pearson's χ²-test. Differences were considered as significant at p<0.05. Data are presented as mean ± SD or as median and inter-quartile range for skewed variables. Univariate correlation analysis was performed by Spearman correlation analysis. Kaplan-Meier time-to-event curves were generated for patients with serum apoA-IV concentrations above and below the median. Multivariable adjusted risk estimates for progression endpoints were calculated using a Cox proportional hazards regression analysis (short Cox regression). A forward likelihood ratio procedure was used to identify variables associated with progression endpoints over time. Verification of the results was done by a backward likelihood ratio procedure.

### 1.5. Limitations of the study

One of the limitations of the present study is the fact that we do not have sequential GFR measurements by iohexol technique during the follow-up period. We felt, however, that it was more important to have an exact measurement of GFR at baseline since a simple measurement of creatinine or a GFR calculation by a formula would have been imprecise. Doubling of serum creatinine is an accepted specific endpoint *⁽⁴⁷⁾*.

### EXAMPLE 2: Univariate association of lipoprotein parameters and progress of CKD

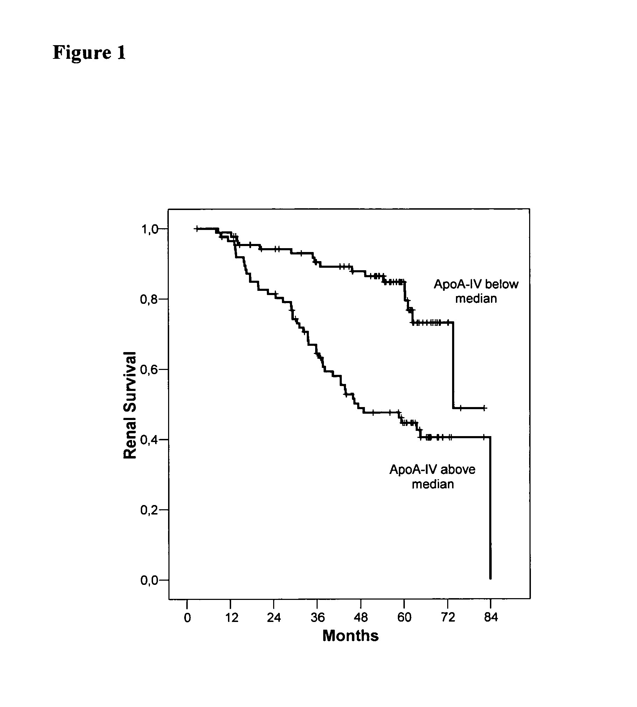

Baseline clinical characteristics and laboratory data of the patients with follow-up are reported in the first data column of Table 1. The median follow-up after completion of the baseline investigation was 53 [3-84] months. During the follow-up 65 patients had progressed to a renal endpoint: 36 patients had doubled their serum creatinine concentration and 29 patients had reached terminal renal failure necessitating renal replacement therapy. Table 1 further summarizes data of patients with and without progression during the follow-up period. Those patients who had reached a progression endpoint were significantly older, had higher baseline serum creatinine levels and protein excretion rates as well as lower GFR and haemoglobin levels. In addition, they had higher triglyceride and apoA-IV concentrations and lower HDL cholesterol levels. Lp(a) was slightly, but not significantly, higher in patients who reached a progression endpoint, but the frequency of low molecular weight (LMW) apo(a) phenotypes was similar in the two patient groups. Analogous results were obtained using univariate Cox regression analysis: GFR and apoA-IV had the strongest influence on progression-free survival (first part of Table 2). When we constructed Kaplan-Meier curves of the progression-free survival comparing patients with supra-median and infra-median (25.9 mg/dL) serum apoA-IV concentrations (Figure 1), patients with the apoA-IV levels above the median had a worse prognosis and significantly faster progression to the endpoint compared to those with apoA-IV levels below the median (log-rank test, p<0.0001): the mean follow-up time to a progression endpoint was 53.7 (95% confidence interval [CI] 47.6-59.8) months compared to 70.0 (95%CI 64.6-75.4) months in the two groups of patients.

**Table 1 shows the baseline clinical and laboratory data of 177 patients with completed follow-up with further stratification of those with and without progression of kidney disease during the follow-up period.**

| | All patients (n=177) | Non-progressors (n=112) | Progressors (n=65) |
|---|---|---|---|
| Gender (male/female) | 118/59 | 74/38 | 44/21 |
| | (67%/33%) | (66%/34%) | (68%/32%) |
| Age (years) | 46.4 ± 12.2 | 44.8 ± 12.6 | 49.1 ± 11.1 *^{a}* |
| Body mass index (kg/m²) | 25.2 ± 3.7 | 24.8 ± 3.5 | 25.7 ± 3.9 |
| Current smokers, n (%) | 34 (19%) | 18 (16%) | 16 (25%) |
| Serum creatinine (mg/dL) | 2.15 ± 1.22 | 1.54 ± 0.61 | 3.21 ± 1.31 |
| Glomerular filtration rate (mL/min/1.73m²) | 64 ± 39 | 79 ± 38 | 38 ± 25 *^{d}* |
| Proteinuria (g/24h/1.73m²) | 1.01 ± 0.92 | 0.87 ± 0.95 | 1.25 ± 0.83 *^{d}* |
| Systolic blood pressure (mmHg) | 137 ± 20 | 136 ± 22 | 137 ± 17 |
| Diastolic blood pressure (mmHg) | 87 ± 13 | 86 ± 14 | 88 ± 12 |
| Pulse pressure (mmHg) | 50 ± 15 | 50 ± 16 | 50 ± 14 |
| Haemoglobin (g/dL) | 13.6 ± 1.8 | 14.1 ± 1.5 | 12.6 ± 1.9 *^{d}* |
| High sensitivity C-reactive protein (mg/L) | 0.28 ± 0.31 | 0.28 ± 0.32 | 0.29 ± 0.31 |
| Total cholesterol (mg/dL) | 216 ± 43 | 215 ± 42 | 217 ± 46 |
| LDL cholesterol (mg/dL) | 138 ± 39 | 137 ± 36 | 138 ± 43 |
| HDL cholesterol (mg/dL) | 44 ± 15 | 46 ± 15 | 40 ± 13 *^{b}* |
| Non-HDL cholesterol (mg/dL) | 172 ± 42 | 169 ± 39 | 177 ± 46 |
| Triglycerides (mg/dL) | 172 ± 95 | 159 ± 93 | 194 ± 96 *^{c}* |
| Lp(a) (mg/dL) | 29 ± 31 | 27 ± 30 | 32 ± 33 |
| LMW apo(a) phenotypes, n (%) | 48 (27%) | 30 (27%) | 18 (28%) |
| ApoA-IV (mg/dL) | 26 ± 8 | 23 ± 8 | 31 ± 6 *^{d}* |
| ApoA-I (mg/dL) | 121 ± 23 | 123 ± 25 | 117 ± 21 |
| ApoB (mg/dL) | 107 ± 26 | 106 ± 26 | 109±27 |
| Use of statins, n (%) | 26 (14.7%) | 16 (14.3%) | 10 (15.4%) |
| Use of fibrates, n (%) | 9 (5.1%) | 6 (5.4%) | 3 (4.6%) |

| | | | |
|---|---|---|---|
| *a:* p<0.05; *b:* p<0.01; *c:* p<0.005; *d:* p<0.001 - comparison between progressors and non-progressors | | | |

### EXAMPLE 3: Multivariate association of lipoprotein parameters and progression of CKD

In order to identify lipoprotein parameters associated with progression over time after adjustment for other variables, we performed a multiple Cox regression analysis. Besides age and gender we added variables to the model which had shown p-values < 0.2 in the univariate Cox regression analysis (first part of Table 2). Only baseline GFR (hazard ratio [HR]=0.714; 95%Cl 0.627-0.814; p<0.0001) and serum apoA-IV concentrations (HR=1.062; 95%CI 1.018-1.108; p=0.006) were significantly associated with progression during the follow-up period. HDL cholesterol and triglycerides did not enter into the model as independent factors irrespective of whether we added these variables as a continuous or categorial variable defined by the median of each variable. ApoA-IV remained in the model (HR=1.045; 95%CI 1.001-1.092; p=0.046) even if we adjusted for asymmetric dimethylarginine (ADMA) which has recently been shown as a significant predictor for progression of kidney disease *⁽¹⁸⁾*.

**Table 2 shows the association of different variables with progression of kidney disease during the observation period using univariate and multiple Cox Proportional Hazards regression models.**

| Variable (increment) | Coefficient | SEM | χ² | HR (95% CI) | p |
|---|---|---|---|---|---|
| **Univariate Cox regression analysis** | | | | | |
| Age (1 year) | 0.021 | 0.011 | 3.419 | 1.021 (0.999-1.045) | 0.064 |
| Gender (0=men, 1=women) | 0.103 | 0.268 | 0.147 | 1.108 (0.656-1.873) | 0.701 |
| GFR (10 mL/min/1.73m²) | -0.389 | 0.059 | 42.976 | 0.678 (0.603-0.761) | <0.0001 |
| 24h-Proteinuria (1g/24h) | 0.264 | 0.121 | 4.742 | 1.302 (1.027-1.652) | 0.029 |
| ApoA-IV (1 mg/dL) | 0.112 | 0.019 | 34.433 | 1.118 (1.077-1.161) | <0.0001 |
| Triglycerides (1 mg/dL) | 0.002 | 0.001 | 2.008 | 1.002 (0.999-1.004) | 0.157 |
| HDL cholesterol (1 mg/dL) | -0.018 | 0.010 | 3.270 | 0.982 (0.964-1.001) | 0.071 |
| Lp(a) (1 mg/dL) | 0.002 | 0.004 | 0.205 | 1.002 (0.994-1.009) | 0.651 |

| **Multiple Cox regression analysis** | | | | | |
|---|---|---|---|---|---|
| GFR (10 mL/min/1.73m²) | -0.336 | 0.066 | 25.627 | 0.714 (0.627-0.814) | <0.0001 |
| ApoA-IV (1 mg/dL) | 0.060 | 0.022 | 7.697 | 1.062 (1.018-1.108) | 0.006 |
| Variables not in the equation: | age (p=0.63), gender (p=0.97), proteinuria (p=0.42), triglycerides (p=0.98), HDL cholesterol (p=0.26) | | | | |

### EXAMPLE 4: ApoE polymorphism and progression CKD

### 4.1. Results

Table 3 shows the distribution of apoE genotypes in renal patients with and without progression to a renal endpoint during the follow-up period. There were no major differences between the two groups with the exception of apoE genotype ε2/ε4. From the six patients showing an ε2/ε4 genotype five progressed to the endpoint. When formally tested against the combined number of other genotype carriers, this difference in frequency reached significance (p=0.027). When we offered a variable describing the ε2/ε4 carrier status as an additional co-variate to the Cox regression model, those with that genotype had a significantly higher probability for a progression of kidney disease (HR=2.68; 95%Cl 1.06-6.75; p=0.037). In this analysis the estimates for GFR and apoA-IV remained almost identical as in the model above.

**Table 3 shows the influence of the apolipoprotien E (apoE) genotype on the progression of kidney disease during the follow-up period in patients with mild and moderate kidney disease. ^{a}**

| **ApoE genotype** | **No progression** | | **Progression** | |
|---|---|---|---|---|
| | n | % | n | % |
| 2/2 | 1 | 0.9 | 1 | 1.5 |
| 2/3 | 17 | 15.5 | 7 | 10.8 |
| 3/3 | 69 | 62.7 | 36 | 55.4 |
| 3/4 | 22 | 20.0 | 15 | 23.1 |
| 4/4 | 0 | 0.0 | 1 | 1.5 |
| 2/4 | 1 | 0.9 | 5 | 7.7 |

| | | | | |
|---|---|---|---|---|
| *^{a}* ApoE genotypes are available in 175 out of 177 patients. | | | | |

### 4. 2. Discussion of ApoE polymorphism

Up to now numerous case-control and cross-sectional studies investigated the apoE polymorphism in kidney patients compared to controls with contrasting results. Only few of them were designed to investigate an association between the apoE polymorphism and the progression of renal insufficiency by a follow-up observation *⁽⁴⁴⁻⁴⁶⁾*. Araki et al. found in Japanese type 2 diabetics that those 31 patients who developed or who showed a progression of nephropathy during an average observation period of 4.4 years were more often ε2 carriers *⁽⁴⁵⁾*. The apo ε4 allele was found to be an independent protective factor for the progression to ESRD in a retrospective follow-up study in Japanese type 2 diabetic patients *⁽⁴⁴⁾*. Results from the prospective ARIC Study in the general population without severe renal dysfunction at baseline recently showed that ε2 moderately increased and ε4 decreased risk of renal disease progression *⁽⁴⁶⁾*. In contrast to these studies, we could not see a negative effect of the ε2 allele or a protective effect of the ε4 allele. When we used the allele-counting method we observed that progressors showed a very similar frequency of the ε2 allele (10.8% vs. 9.1%) and a higher rather than a lower frequency of the ε4 allele (16.9% vs. 10.8%) which did not reach significance, however. It is not clear whether this finding can be explained by differences in study design, patient selection or definition of kidney disease progression. The above mentioned prospective studies either investigated patients with type 2 diabetes mellitus considering albuminuria or proteinuria as progression endpoint *⁽⁴⁵⁾* or focused on subjects at the population level without kidney disease at baseline and an endpoint mostly defined by an increase in creatinine of at least 0.4 mg/dL *⁽⁴⁶⁾.* Our study, however, included patients with primary kidney disease at study entry. Progression was defined as doubling of baseline serum creatinine or necessity of renal replacement therapy during follow-up. The only noticeable observation concerning the apoE polymorphism in our study was that five of the six carriers of apoE genotype ε2/ε4 experienced a progression of the disease. However, we are aware of the low frequency of this genotype and of the need that the finding is confirmed by future large studies.

### EXAMPLE 5: Further discussion

### 5.1. Lipid and lipoprotein profile and progression of kidney disease

There is little doubt that kidney disease is associated with abnormalities in lipoprotein metabolism. As reviewed in Table 4 it has remained controversial, however, whether dyslipidemia plays an independent role in the progression of kidney diseases. The basic pathophysiological concept is derived from the hypothesis that dyslipidemia, among other factors, causes glomerular injury leading to glomerulosclerosis *⁽¹⁹⁾*. It has been suggested that glomerulosclerosis and atherosclerosis share common pathophysiological pathways *⁽²⁰⁾.* However, little is known about the exact mechanism *⁽²¹⁾.*

This prospective study had a follow-up time of adequate duration, used exact measurements of GFR and primary endpoints were reached by a third of the participants. No significant association was found between baseline classical lipid or lipoprotein parameters and the progression of renal insufficiency. These results are consistent with another prospective study in 138 patients with moderate renal insufficiency *⁽⁸⁾*. Locatelli et al. *⁽⁵⁾* also found no correlation between the baseline lipid profile and the endpoint of end-stage renal disease (ESRD) in 311 non-diabetic, non-nephrotic patients with moderate renal insufficiency followed for two years. On the other hand Samuelsson et al. *⁽⁷⁾* described in 73 patients with mild to severe renal insufficiency total and LDL cholesterol as well as apoB to be significantly associated with a rapid decline in renal function but not triglycerides or HDL cholesterol. The Modification of Diet in Renal Disease (MDRD) Study demonstrated lower HDL cholesterol levels as independent predictor of a decline in GFR in kidney patients; however, triglyceride levels were not measured *⁽⁶⁾*. In our study lower HDL cholesterol showed only a borderline association with progression of disease which was no longer significant after adjusting for GFR in the multiple Cox regression analysis. Syrjanen et al. *⁽¹¹⁾* found hypertriglyceridemia as an independent risk factor for progression of IgA nephropathy.

These nonuniform data illustrate that so far it has remained uncertain which lipoproteins are the best predictors for a progression of kidney disease (Table 4). The finding of low HDL cholesterol levels as predictor of progression is in line with studies performed in the general population *^{(4,22)}*. The Atherosclerosis Risk in Communities (ARIC) study reported that higher triglyceride levels and lower HDL cholesterol were associated with a higher relative risk of worsening renal function over three years *⁽⁴⁾*. The prospective, randomized Helsinki Heart Study investigated dyslipidemic subjects with non-HDL cholesterol above 200 mg/dL who were at baseline free of renal disease and found lower HDL cholesterol and an elevated LDL/HDL cholesterol ratio to be independent predictors of an increase of serum creatinine during study follow-up; LDL cholesterol and triglycerides were not independent predictors. However, these variables explained only a very small fraction (at best 1%) of the variance in creatinine increase *⁽²²⁾*. Such small contribution can only be detected in large studies. Further explanations for differences to our and other studies in kidney patients might be that studies in the general population investigated individuals without primary kidney disease at baseline or with mild impairment of kidney function at most. Another explanation might be the definition of the progression endpoint which was only a minor increase in serum creatinine in some of these studies *⁽⁴⁾*.

Even if the evidence for an association between lipid parameters and the progression of renal disease in humans is not consistent, several studies investigated the effect of lipid-lowering on the progression of renal insufficiency. In a number of animal models, lipogenic diets worsen, while cholesterol-lowering medications ameliorate renal injury *⁽²³⁻²¹⁾*. Fried et al. performed a meta-analysis that examined the role of lipid-lowering therapy on renal function in humans *⁽²⁸⁾.* The data were derived from lipid-lowering trials that commonly performed post-hoc analysis of subgroups with renal insufficiency for renal endpoints that were not predefined. They suggested that lipid-lowering therapy may slow progression but this conclusion was derived from few trials with small numbers and generally short follow-up times *⁽²⁸⁾*. In case these agents show indeed a progression-retarding effect, it remains to be proven whether this retardation derives from lipid-lowering or from pleiotropic effects of statins.

It is also interesting to note that Lp(a) concentrations were not predictive for the progression of kidney disease although in vivo experiments pointed to a progression-advancing effect. Atherogenic lipoproteins such as LDL cholesterol or Lp(a), especially when oxidized, induce the formation of oxygen radicals in arteries, in glomeruli and in juxtaglomerular cells which results in an inhibition of nitric oxide-mediated vasodilatation ^{*(29*)}, stimulation of renin release and modulation of mesangial cell growth and apoptosis *⁽³⁰⁻³³⁾*. However, Lp(a) concentrations in our and two other studies *^{(8,34)}* were not related to the progression of kidney disease.

### 5.2. Apolipoprotein A-IV

This study was the first to investigate the association between a novel lipid parameter, i.e. apoA-IV concentration and progression of renal insufficiency. We found that higher baseline plasma apoA-IV concentrations were one of the best predictors for the progression of kidney disease apart from baseline GFR. This association was independent from other lipoprotein parameters, from asymmetric dimethylarginine (ADMA) recently shown to be a highly significant predictor of kidney disease progression *⁽¹⁸⁾* as well as independent from proteinuria and blood pressure. However, this does not necessarily imply that apoA-IV *per se* is pathogenic. It is conceivable that apoA-IV reflects catabolism of this apolipoprotein in the kidney not entirely reflected by the changes in GFR. It is known that ESRD affects apoA-IV serum concentrations. We and others reported a pronounced increase of apoA-IV concentrations in hemodialysis patients *⁽³⁵⁻³⁸⁾* We could even demonstrate that in patients with kidney disease a significant increase in apoA-IV concentrations is found even when GFR is still within the normal range *⁽¹⁵⁾*. It therefore seems that apoA-IV is an early marker of renal impairment. This is supported by our recent findings of apoA-IV immunoreactivity in kidney tubular cells suggesting a direct role of the human kidney in apoA-IV metabolism *⁽³⁹⁾*. A granular staining pattern in those cells probably represents lysosomes degrading apoA-IV. Interestingly, a high correlation was found between apoA-IV and GFR (r=-0.62) *⁽¹⁵⁾*. If we compare GFR and apoA-IV in the univariate Cox regression model, both parameters predict similarly the progression-free survival (χ² of 43 vs. 34, see Table 2). Nevertheless, apoA-IV still significantly contributes to the prediction of disease progression after adjustment for GFR which supports the additional value of apoA-IV as kidney function parameter.

The association of higher apoA-IV levels with progression of kidney disease is unexpected, given the physiological functions of apoA-IV. According to in vitro studies apoA-IV participates in several steps of the reverse cholesterol transport pathway, which removes cholesterol from peripheral cells and directs it to liver and steroidogenic organs for metabolism *⁽⁴⁰⁻⁴²⁾.* Furthermore, other studies documented antioxidative properties of apoA-IV ^{*(43*)}. One might therefore have anticipated that the increased apoA-IV levels in impaired kidney function would have resulted in improved removal of cholesterol from mesangial cells and, together with the antioxidative properties, slower progression. The observation of an opposite effect would be compatible with the assumption that apoA-IV is not fully functionally active or that high apoA-IV levels reflect an aspect of renal impairment which does not go parallel to GFR.

**Table 4 shows a summary of prospective studies in non-diabetic kidney patients examining the relationship of dyslipidemia and the progression of kidney disease. (The table lists mostly adjusted results unless otherwise stated.)**

| **Author, Year** | **Patients** | **Duration of follow-up** | **Definition of progression** | **Associations with progression of kidney disease** |
|---|---|---|---|---|
| Hunsicker et al., 1997 *⁽⁶⁾* | n=840; | Up to 3.5 yrs | rate of decline in GFR (GFR slope mL/min/year) | - lower HDL-C independent predictor of a faster decline in GFR |
| | GFR 15-55 mL/min/1.73m² | | | |
| | | | | - no significant effect by TC, LDL-C, apoA-1, apoB |
| Locatelli et al., 1996 *⁽⁵⁾* | n=311; | 2 yrs | need for dialysis, doubling of baseline Cr | - no correlation with TC |
| | Cr 119-619 mmol/L and CCr <60 mL/min | | | |
| Syrjanen et al., 2000 *⁽¹¹⁾* | n=223 patients with IgA nephropathy; | median 10 yrs (0.2 - 17 yrs) | Cr > 125 µmol/L (men) or > 105 µmol/L (women) and > 20% elevation from baseline | - hypertriglyceridemia independent risk factor for progression of disease |
| | 81% normal Cr levels, 19% elevated Cr levels | | | - no significant correlation with TC, LDL-C, HDL-C, LDL-C/HDL-C |
| Massy et al., 1999 *⁽⁸⁾* | n=138 (including 2-3% patients with T2DM); | mean about 7 yrs | end-stage renal disease | - no correlation with TG, HDL-C, TC, apoA-I, apo-B, Lp(a) |
| | CCr 20-70 mL/min | | | |
| Washio et al., 1996 *⁽¹⁰⁾* | n=104 (including 9 patients with T2DM); Cr 0.5-5.0 mg/dL | mean 4.1 yrs | (-1) x (the slope of the 1/Cr plotted against the observation time) | - positive correlation with TC level |
| | | | | - no significant correlation with TG |
| Samuelsson et al., 1997 *⁽⁷⁾* | n=73; GFR 15-75 mL/min/1.73 m² | mean 3.2 yrs | annual change in GFR | - significant association with TC, LDL-C and apoB (in univariate analysis) |
| | | | | - no significant relation with TG, HDL-C, VLDL-C, apoA-I, apoA-II, apoC-III, apoE |
| Cappelli et al., 1992 *⁽⁹⁾* | n=52; CCr 20-60 mL/min | 12 months | slope of the regression line of 1/Cr negative at end of follow-up period | - significant relationship with apoA/apoB ratio |
| | | | | - no association with TC, HDL-C, LDL-C, VLDL-C, TG, apoB |
| Mazouz et al., 1999 *⁽¹²⁾* | n=49 (including 7 patients with T2DM); | at least 2 yrs before the start of dialysis *^{a}* | delta Ccr | - no significant association with TC and TG |
| | mean CCr 24 ± 14 mL/min | | | |

| | | | | |
|---|---|---|---|---|
| *^{a}* 49 patients selected out of 173 presently treated HD patients on the basis of availability of a quarterly follow-up for 2 years before starting dialysis. Cr, creatinine; CCr, creatinine clearance; T2DM, type 2 diabetes mellitus; TC, total cholesterol; LDL-C, LDL cholesterol; HDL-C, HDL cholesterol; TG, triglycerides; apo, apolipoprotein | | | | |

### References

1. Moorhead JF, Chan MK, E1 Nahas M, Varghese Z: Lipid nephrotoxicity in chronic progressive glomerular and tubulo-interstitial disease. Lancet 2:1309-1311, 1982
2. Keane WF: Lipids and the kidney. Kidney Int 46:910-920, 1994
3. Keane WF: The role of lipids in renal disease: future challenges. Kidney Int 57 Suppl 75:S27-S31, 2000
4. Muntner P, Coresh J, Smith JC, Eckfeldt J, Klag MJ: Plasma lipids and risk of developing renal dysfunction: the atherosclerosis risk in communities study. Kidney Int 58:293-301,2000
5. Locatelli F, Marcelli D, Comelli M, Alberti D, Graziani G, Buccianti G, Redaelli B, Giangrande A, the Northern Italian Cooperative Study Group: Proteinuria and blood pressure as causal components of progression to end-stage renal failure. Nephrol Dial Transplant 11:461-467, 1996
6. Hunsicker LG, Adler S, Caggiula A, England BK, Greene T, Kusek JW, Rogers NL, Teschan PE: Predictors of the progression of renal disease in the Modification of Diet in Renal Disease Study. Kidney Int 51:1908-1919, 1997
7. Samuelsson O, Mulec H, Knight-Gibson C, Attman PO, Kron B, Larsson R, Weiss L, Wedel H, Alaupovic P: Lipoprotein abnormalities are associated with increased rate of progression of human chronic renal insufficiency. Nephrol Dial Transplant 12:1908-1915, 1997
8. Massy ZA, Khoa TN, Lacour B, Descamps-Latscha B, Man NK, Jungers P: Dyslipidaemia and the progression of renal disease in chronic renal failure patients. Nephrol Dial Transplant 14:2392-2397, 1999
9. Cappelli P, Evangelista M, Bonomini M, Palmieri PF, Albertazzi A: Lipids in the progression of chronic renal failure. Nephron 62:31-35, 1992
10. Washio M, Okuda S, Ikeda M, Hirakata H, Nanishi F, Onoyama K, Yoshimura T, Fujishima M: Hypercholesterolemia and the progression of the renal dysfunction in chronic renal failure patients. J Epidemiol 6:172-177, 1996
11. Syrjanen J, Mustonen J, Pasternack A: Hypertriglyceridaemia and hyperuricaemia are risk factors for progression of IgA nephropathy. Nephrol Dial Transplant 15:34-42, 2000
12. Mazouz H, Kacso I, Ghazali A, El Esper N, Moriniere P, Makdassi R, Hardy P, Westeel PF, Achard JM, Pruna A, Fournier A: Risk factors of renal failure progression two years prior to dialysisis. Clin Nephrol 51:355-366, 1999
13. Crook ED, Thallapureddy A, Migdal S, Flack JM, Greene EL, Salahudeen A, Tucker JK, Taylor HA, Jr.: Lipid abnormalities and renal disease: is dyslipidemia a predictor of progression of renal disease? Am J Med Sci 325:340-348, 2003
14. Kronenberg F, Kuen E, Ritz E, Junker R, König P, Kraatz G, Lhotta K, Mann JFE, Müller GA, Neyer U, Riegel W, Riegler P, Schwenger V, Von Eckardstein A: Lipoprotein(a) serum concentrations and apolipoprotein(a) phenotypes in mild and moderate renal failure. J Am Soc Nephrol 11:105-115, 2000
15. Kronenberg F, Kuen E, Ritz E, Junker R, König P, Kraatz G, Lhotta K, Mann JFE, Müller GA, Neyer U, Riegel W, Riegler P, Schwenger V, Von Eckardstein A: Apolipoprotein A-IV serum concentrations are elevated in mild and moderate renal failure. J Am Soc Nephrol 13:461-469, 2002
16. Kronenberg F, Lobentanz E-M, König P, Utermann G, Dieplinger H: Effect of sample storage on the measurement of lipoprotein(a), apolipoproteins B and A-IV, total and high-density lipoprotein cholesterol and triglycerides. J Lipid Res 35:1318-1328, 1994
17. Bostom AG, Kronenberg F, Ritz E: Predicitive performance of renal function equations for patients with chronic kidney disease and normal serum creatinine levels. J Am Soc Nephrol 13:2140-2144, 2002
18. Fliser D, Kronenberg F, Kielstein JT, Morath C, Bode-Böger SM, Haller H, Ritz E, for the MMKD Study Group: Asymmetric dimethylarginine (ADMA) and progression of chronic kidney disease: The Mild to Moderate Kidney Disease (MMKD) Study. J Am Soc Nephrol (in press)
19. Diamond JR: Analogous pathobiologic mechanisms in glomerulosclerosis and atherosclerosis. Kidney Int 39 Suppl. 31:S29-S34, 1991
20. Kasiske BL: Relationship between vascular disease and age-associated changes in the human kidney. Kidney Int 31:1153-1159, 1987
21. Diamond JR, Karnovsky MJ: A putative role of hypercholesterolemia in progressive glomerular injury. Annu Rev Med 43:83-92, 1992
22. Manttari M, Tiula E, Alikoski T, Manninen V: Effects of hypertension and dyslipidemia on the decline in renal function. Hypertension 26:670-675, 1995
23. Kasiske BL, O'Donnell MP, Schmitz PG, Kim Y, Keane WF: Renal injury of diet-induced hypercholesterolemia in rats. Kidney Int 37:880-891, 1990
24. Wellmann KF, Volk BW: Renal changes in experimental hypercholesterolemia in normal and in subdiabetic rabbits. II. Long term studies. Lab Invest 24:144-155, 1971
25. Kasiske BL, O'Donnell MP, Keane WF: Pharmacological treatment of hyperlipidemia reduces glomerular injury in the rat 5/6 nephrectomy model of chronic renal failure. Circ Res 62:367-374, 1988
26. Kasiske BL: Risk factors for accelerated atherosclerosis in renal transplant recipients. Am J Med 84:985-992, 1988
27. Harris KPG, Purkerson ML, Yates J, Klahr S: Lovastatin ameliorates the development of glomerulosclerosis and uremia in experimental nephrotic syndrome. Am J Kidney Dis 15:16-23, 1990
28. Fried LF, Orchard TJ, Kasiske BL: Effect of lipid reduction on the progression of renal disease: A meta-analysis. Kidney Int 59:260-269, 2001
29. Galle J, Bengen J, Schollmeyer P, Wanner C: Impairment of endothelium-dependent dilation in rabbit renal arteries by oxidized lipoprotein(a) - Role of oxygen-derived radicals. Circulation 92:1582-1589, 1995
30. Wanner C, Greiber S, Krämer-Guth A, Heinloth A, Galle J: Lipids and progression of renal disease: Role of modified low density lipoprotein and lipoprotein(a). Kidney Int 52 Suppl. 63: S102-S106, 1997
31. Greiber S, Krämer-Guth A, Pavenstädt H, Gutenkunst M, Schollmeyer P, Wanner C: Effects of lipoprotein(a) on mesangial cell proliferation and viability. Nephrol Dial Transplant 11:778-785, 1996
32. Mondorf UF, Piiper A, Herrero M, Olbrich HG, Bender M, Gross W, Scheuermann E, Geiger H: Lipoprotein(a) stimulates growth of human mesangial cells and induces activation of phospholipase C via pertussis toxin-sensitive G proteins. Kidney Int 55:1359-1366, 1999
33. Galle J, Schneider R, Heinloth A, Wanner C, Galle PR, Conzelmann E, Dimmeler S, Heermeier K: Lp(a) and LDL induce apoptosis in human endothelial cells and in rabbit aorta: role of oxidative stress. Kidney Int 55:1450-1461, 1999
34. Samuelsson O, Attman P-O, Knight-Gibson C, Larsson R, Mulec H, Wedel H, Weiss L, Alaupovic P: Plasma levels of lipoprotein(a) do not predict progression of human chronic renal failure. Nephrol Dial Transplant 11:2237-2243, 1996
35. Nestel P, Noakes M, Belling B, McArthur R, Clifton P, Janus E, Abbey M: Plasma lipoprotein lipid and Lp[a] changes with substitution of elaidic acid for oleic acid in the diet. J Lipid Res 33:1029-1036, 1992
36. Seishima M, Muto Y: An increased apo A-IV serum concentration of patients with chronic renal failure on hemodialysis. Clin Chim Acta 167:303-311, 1987
37. Dieplinger H, Lobentanz E-M, König P, Graf H, Sandholzer C, Matthys E, Rosseneu M, Utermann G: Plasma apolipoprotein A-IV metabolism in patients with chronic renal disease. Eur J Clin Invest 22:166-174, 1992
38. Kronenberg F, König P, Neyer U, Auinger M, Pribasnig A, Lang U, Reitinger J, Pinter G, Utermann G, Dieplinger H: Multicenter study of lipoprotein(a) and apolipoprotein(a) phenotypes in patients with end-stage renal disease treated by hemodialysis or continuous ambulatory peritoneal dialysis. J Am Soc Nephrol 6:110-120, 1995
39. Haiman M, Salvenmoser W, Scheiber K, Lingenhel A, Rudolph C, Schmitz G, Kronenberg F, Dieplinger H: Immunohistochemical localization of apolipoprotein A-IV in human kidney tissue. Kidney Int (in press).
40. Stein O, Stein Y, Lefevre M, Roheim PS: The role of apolipoprotein A-IV in reverse cholesterol transport studied with cultured cells and liposomes derived from another analog of phosphatidylcholine. Biochim Biophys Acta 878:7-13, 1986
41. Dvorin E, Gorder NL, Benson DM, Gotto Jr. AM: Apolipoprotein A-IV. A determinant for binding and uptake of high density lipoproteins by rat hepatocytes. J Biol Chem 261:15714-15718, 1986
42. Steinmetz A, Barbaras R, Ghalim N, Clavey V, Fruchart J-C, Ailhaud G: Human apolipoprotein A-IV binds to apolipoprotein A-I/A- II receptor sites and promotes cholesterol efflux from adipose cells. J Biol Chem 265:7859-7863, 1990
43. Qin XF, Swertfeger DK, Zheng SQ, Hui DY, Tso P: Apolipoprotein AIV: A potent endogenous inhibitor of lipid oxidation. Am J Physiol 274:H1836-H1840, 1998
44. Kimura H, Suzuki Y, Gejyo F, Karasawa R, Miyazaki R, Suzuki S, Arakawa M: Apolipoprotein E4 reduces risk of diabetic nephropathy in patients with NIDDM. Am J Kidney Dis 31:666-673, 1998
45. Araki S, Koya D, Makiishi T, Sugimoto T, Isono M, Kikkawa R, Kashiwagi A, Haneda M: APOE polymorphism and the progression of diabetic nephropathy in Japanese subjects with type 2 diabetes: results of a prospective observational follow-up study. Diabetes Care 26:2416-2420, 2003
46. Hsu CC, Kao WH, Coresh J, Pankow JS, Marsh-Manzi J, Boerwinkle E, Bray MS: Apolipoprotein E and progression of chronic kidney disease. JAMA 293:2892-2899, 2005
47. Rossing P: Doubling of serum creatinine: is it sensitive and relevant? [editorial]. Nephrol Dial Transplant 13:244-246, 1998

## Claims

1. An *in vitro* method for predicting chronic kidney disease progression in a subject to be examined, wherein the method comprises determination of apolipoprotein A-IV (apoA-IV) in a bodily fluid sample taken from the subject to be examined, and wherein
- the ApoA-IV concentration is compared to a reference value determined from non-progressing patients afflicted with mild to moderate chronic kidney disease, and
- wherein an elevation of the ApoA-IV concentration is a predictor of disease progression.

2. The method according to claim 1, wherein the chronic kidney disease is a non-diabetic kidney disease, preferably developed from a primary kidney disease selected from the group consisting of glomerulonephritis, adult polycystic kidney disease, and interstitial nephritis.

3. The method according to claim 1, wherein the chronic kidney disease is a diabetic kidney disease, preferably diabetic nephropathy.

4. The method according to any of the preceding claims, wherein the determination of ApoA-IV is a quantitative measurement of ApoA-IV concentration, preferably determined in serum by use of an ELISA.

5. The method according to any of claims 2 to 4, wherein the elevation of the ApoA-IV concentration is preferably an elevation of at least 3 mg/dl, more preferably an elevation of at least 5 mg/dl, and most preferably an elevation of at least 10 mg/dl.

6. The method according to any of the preceding claims, further comprising a determination of the glomerular filtration rate, preferably determined by use of the iothalamate clearance technique.

7. The method according to claim 6, wherein a reduction of the glomerular filtration rate is a predictor of disease progression, preferably a reduction of at least 10 ml/min/1.73 m².

8. *In vitro* use of ApoA-IV as a predictor of chronic kidney disease progression according to claim 1.

9. The use according to claim 8, wherein the disease is a non-diabetic chronic kidney disease, preferably developed from a primary kidney disease, selected from the group consisting of glomerulonephritis, adult polycystic kidney disease, and interstitial nephritis.

10. The use according to claim 8, wherein the disease is a diabetic chronic kidney disease.

11. The use according to any of claims 8 to 10, wherein ApoA-IV is used together with the glomerular filtration rate as predictors of chronic kidney disease progression.

## Patentansprüche

1. *In vitro* Verfahren zur Vorhersage der Progression einer chronischen Nierenerkrankung eines zu untersuchenden Probanden, wobei das Verfahren den Schritt einschließt, Apolipoprotein A-IV (ApoA-IV) in einer Körperflüssigkeitsprobe, die von dem Probanden erhalten wurde, zu bestimmen, und wobei
- die ApoA-IV-Konzentration mit einem Referenzwert verglichen wird, der von nicht-progredienten Patienten mit leichter bis moderater chronischer Nierenerkrankung bestimmt wird, und
- wobei eine Erhöhung der ApoA-IV-Konzentration ein Indikator für die Progression der Erkrankung ist.

2. Verfahren nach Anspruch 1, wobei die chronische Nierenerkrankung eine nicht-diabetische Nierenerkrankung ist, vorzugsweise eine, die sich aus einer primären Nierenerkrankung entwickelt hat, ausgewählt aus der Gruppe bestehend aus Glomerulonephritis, polyzystischer Nierenerkrankung und interstitieller Nephritis.

3. Verfahren nach Anspruch 1, wobei die chronische Nierenerkrankung eine diabetische Nierenerkrankung ist, vorzugsweise diabetische Nephropathie.

4. Verfahren nach einem der vorhergehenden Ansprüche, wobei die Bestimmung des ApoA-IV eine quantitative Messung der ApoA-IV-Konzentration ist, die vorzugsweise aus Serum unter Verwendung eines ELISA bestimmt wird.

5. Verfahren nach einem der Ansprüche 2 bis 4, wobei eine Erhöhung der ApoA-IV-Konzentration vorzugsweise eine Erhöhung von mindestens 3 mg/dl, weiter bevorzugt eine Erhöhung von mindestens 5 mg/dl und am meisten bevorzugt eine Erhöhung von mindestens 10 mg/dl ist.

6. Verfahren nach einem der vorhergehenden Ansprüche, weiterhin umfassend eine Bestimmung der glomerulären Filtrationsrate, vorzugsweise durch Verwendung der lothalamat-Clearance Technik.

7. Verfahren nach Anspruch 6, wobei eine Reduktion der glomerulären Filtrationsrate ein Indikator für die Progression der Krankheit ist, vorzugsweise eine Reduktion um mindestens 10 ml/min/1,73 m².

8. *In-vitro* Verwendung von ApoA-IV als Prädiktor der Progression der chronischen Nierenerkrankung nach Anspruch 1.

9. Verwendung nach Anspruch 8, wobei die Krankheit eine nicht-diabetische chronischen Nierenerkrankung ist, vorzugsweise eine, die sich aus einer primären Nierenerkrankung entwickelt hat, ausgewählt aus der Gruppe bestehend aus Glomerulonephritis, polyzystischer Nierenerkrankung und interstitieller Nephritis.

10. Verwendung nach Anspruch 8, wobei die Krankheit eine diabetische chronische Nierenerkrankung ist.

11. Verwendung nach einem der Ansprüche 8 bis 10, bei dem ApoA-IV zusammen mit der glomerulären Filtrationsrate als Prädiktoren für die Progression der chronischen Nierenerkrankung verwendet wird.

## Revendications

1. Procédé *in vitro* pour la prédiction de la progression d'une maladie rénale chronique chez un sujet à examiner, dans lequel le procédé comprend la détermination de l'apolipoprotéine A-IV (ApoA-IV) dans un échantillon de fluide corporel prélevé chez le sujet à examiner, et dans lequel
- la concentration ApoA-IV est comparée à une valeur de référence déterminée de patients non progressifs souffrant d'une maladie rénale chronique légère à modérée,
et
- dans lequel une élévation de la concentration d'ApoA-IV est un indicateur de la progression de la maladie.

2. Procédé selon la revendication 1, dans laquelle la maladie rénale chronique est une maladie rénale non-diabétique, de préférence développé d'une maladie rénale primaire choisi parmi la glomérulonéphrite, l'adulte polykystose rénale, et la néphrite interstitielle.

3. Procédé selon la revendication 1, dans laquelle la maladie rénale chronique est une maladie rénale diabétique, de préférence la néphropathie diabétique.

4. Procédé selon l'une quelconque des revendications précédentes, dans lequel la détermination de l'ApoA-IV est une mesure quantitative de la concentration d'ApoA-IV, de préférence déterminé dans le sérum en utilisant un ELISA.

5. Procédé selon l'une quelconque des revendications 2 à 4, dans lequel une élévation de la concentration d'ApoA-IV est de préférence une hauteur d'au moins 3 mg / dl, plus préférablement une élévation d'au moins 5 mg / dl, et le plus préférablement une élévation d'au moins 10 mg / dl.

6. Procédé selon l'une quelconque des revendications précédentes, comprenant en outre une détermination du débit de filtration glomérulaire, de préférence déterminé à l'aide de la technique de clairance de l'iothalamate.

7. Procédé selon la revendication 6, dans lequel une réduction du débit de filtration glomérulaire est un indicateur de la progression de la maladie, de préférence une réduction d'au moins 10 ml/min/1,73m².

8. Utilisation *in vitro* d'ApoA-IV en tant que prédicteur de la progression de la maladie rénale chronique selon la revendication 1.

9. Utilisation selon la revendication 8, dans laquelle la maladie est une maladie rénale chronique non diabétique, de préférence développé d'une maladie rénale primaire choisi dans le groupe constitué par la glomérulonéphrite, l'adulte polykystose rénale, et la néphrite interstitielle.

10. Utilisation selon la revendication 8, dans laquelle la maladie est une maladie rénale chronique diabétique.

11. Utilisation selon l'une quelconque des revendications 8 à 10, dans lequel l'ApoA-IV est utilisé en même temps que le débit de filtration glomérulaire comme prédicteurs de la progression de la maladie rénale chronique.
